# EUROPEAN PATENT APPLICATION

(11) **EP 1 433 450 A1**
(43) Date of publication of application: **30.06.2004**
(21) Application number: 02028909.6
(22) Date of filing: 23.12.2002
(51) Int. Cl.: A61F 13/02, A61L 15/24, C08K 5/00, C08J 5/18

(54) **Polymeric compositions for moisture vapour permeable structures with improved structural stability and structures comprising said compositions**

(71) Applicant: THE PROCTER & GAMBLE COMPANY, Cincinnati, Ohio 45202 (US)
(72) Inventor: Gonzales, Denis Alfred, 65123 Pescara (IT); Macbeath, Calum, 66023 Francavilla al Mare (Chieti) (IT); Lunetto, Pietro, Manoppello (Pescara) (IT); Spina, Enrico, 65100 Pescara (IT)
(74) Representative: Veronese, Pancrazio

(57) **Abstract**

The present invention relates to hydrophilic polymeric compositions for making a liquid impermeable moisture vapour permeable structure, for example by forming the composition into a layer or coating onto a suitable substrate for example a microporous film and to composite structures comprising the compositions. The hydrophilic polymeric compositions of the present invention do not solidify at room temperature, but remain viscous or "creamy". This prevents layers made from the hydrophilic polymeric composition of the present invention from settling as a film so that delamination of a composite structure comprising the hydrophilic polymeric composition coated into a layer onto a suitable substrate is avoided. The hydrophilic polymeric compositions of he present invention can find a variety of applications wherein liquid imperviousness and moisture vapour permeability are desirable.

## Description

### Field of the Invention

The present invention relates to polymeric compositions for making a moisture vapour permeable, liquid impermeable structure, for example by forming a moisture vapour permeable, liquid impermeable composite structure where a layer of said polymeric compositions is coated onto a substrate. The compositions of the present invention can find a variety of applications wherein moisture vapour permeability and water imperviousness are desirable.

### Background of the Invention

Thermoplastic films which provide a liquid barrier in addition to providing moisture vapour permeability are known in the art. Particularly preferred are hydrophilic continuous films that do not allow the flow of moisture vapour through open pores or apertures in the material, but do transfer substantial amounts of moisture vapour through the film by absorbing water on one side of the film where the moisture vapour concentration is higher, and desorbing or evaporating it on the opposite side of the film where the moisture vapour concentration is lower. Such films are typically formed from a thermoplastic hydrophilic polymer or from a thermoplastic polymeric composition comprising a thermoplastic hydrophilic polymer, or a blend of thermoplastic hydrophilic polymers. Thermoplastic hydrophilic polymers and thermoplastic hydrophilic polymeric compositions having the above described characteristics are also known in the art as "monolithic polymers" and "monolithic compositions", and the moisture vapour permeable, liquid impermeable layers or films made therefrom are known as "monolithic layers" or "monolithic films". They can be used as such, or incorporated in laminated structures.

For example WO 95/16746 discloses films prepared from mixtures of a) block copolyether ester, block copolyether amides (e.g. Pebax™) and/or polyurethane and b) thermoplastic polymer which is incompatible with a, and c) a compatibiliser. The films are liquid impermeable and have moisture vapour permeability of about 700 g/m² day. Also, US 5,447,783 discloses a vapour permeable water resistant multi component film structure having at least three layers. The outer layers are hydrophobic copolyetherester elastomers having a thickness of 1.3-7.6 micrometers and a WVTR of 400-2500 g/m² 24h and the inner layer is a hydrophilic copolyetherester elastomer having a thickness of 7.6 - 152 micrometers and a WVTR of at least 3500 g/m² 24h.

US 5,445,875 discloses a waterproof, bloodproof and virusproof breathable laminate. The laminate comprises a woven/nonwoven fabric and an extruded film such as Hytrel^{TM} having a thickness of about 1 mil (25.4 micrometers).

US 5,532,053 discloses a high moisture transmission medical film which can be laminated onto a nonwoven material. The laminate film comprises a first layer of polyether ester copolymer and second and third layers selected from a specified group of polymers. The film has a MVTR of greater than 750 g/m² 24h (ASTM F1249) and a thickness of less than 1 mil (25.4 micrometer) preferably 0.6 mil to 0.75 mil (15 -19 micrometers).

US 4,938,752 discloses absorbent articles comprising films of copolyether esters which have reduced water permeability, a water vapour permeability of 500 g/m² 24h (as measured in a specified described test) and a thickness of 5-35 micrometers. There is no disclosure of a supportive substrate.

US 4,493,870 discloses a flexible layered waterproof product comprising a textile material covered with a film of a copolyetherester having an MVTR of at least 1000 g/m² 24h (ASTM E96- 66) having a thickness of 5 to 35 micrometers.

GB 2024100 discloses a flexible layered water resistant article comprising a microporous hydrophobic outer layer which is moisture vapour permeable but resist liquids and a hydrophilic inner layer of polyetherpolyurethane having a MVTR of above 1000 g/m² 24h.

In our patent applications WO 99/64077 entitled "Low viscosity thermoplastic compositions for moisture vapour permeable structures and the utilisation thereof in absorbent articles", and WO 99/64505 entitled "Low viscosity thermoplastic compositions for structures with enhanced moisture vapour permeability and the utilisation thereof in absorbent articles", thermoplastic hydrophilic polymeric compositions comprising a thermoplastic hydrophilic polymer, or a blend of thermoplastic hydrophilic polymers, are disclosed for making hydrophilic continuous moisture vapour permeable, liquid impermeable films or layers having preferred characteristics of moisture vapour permeability and liquid imperviousness. The disclosed preferred thermoplastic hydrophilic polymeric compositions are also readily processable so as to provide a coating having the desired thickness onto a substrate, so avoiding the need of complex traditional extrusion apparatuses. This is achieved by modifying the viscosity of the thermoplastic hydrophilic polymers by means of the inclusion in the composition of a suitable plasticiser or blend of plasticisers that lowers such viscosity. This allows to utilise with these preferred compositions typical process conditions known in the art for the direct coating of low viscosity hot melt compositions onto a substrate in order to form a moisture vapour permeable, liquid impervious film or layer.

Particularly preferred hydrophilic plasticisers are described in WO 99/64505, which, in addition to adjusting the viscosity of the compositions, also provide the thermoplastic hydrophilic polymeric compositions with a further benefit in terms of moisture vapour permeability.

As shown in the above cited prior art documents, a preferred use of known thermoplastic hydrophilic polymers and thermoplastic hydrophilic polymeric compositions ("monolithic polymers" and "monolithic compositions") for making moisture vapour permeable, liquid impermeable layers, is in the manufacture of moisture vapour permeable, liquid impermeable composite structures wherein one or more layers of the thermoplastic hydrophilic polymer or thermoplastic hydrophilic polymeric composition are connected to one or more different substrates, for example a fibrous layer, such as a nonwoven fabric, or a microporous film.

However, this type of construction suffers from some disadvantages, depending on the bonding mean used to achieve said connection between said substrate and said layer of thermoplastic hydrophilic polymer or polymeric composition. For example said connection can be achieved by using an adhesive, or by means of heat and pressure, such as for example by heat bonding. These known methods are however not preferred since, for example, bonding by means of adhesive implies the addition of a further layer to the laminate structure, which may not be desirable in terms of cost, ease of manufacture, and of overall breathability of the resulting structure. Means involving heat and pressure on the other hand can be detrimental for the integrity of the layer formed from the hydrophilic polymeric composition, possibly modifying its moisture vapour permeability, and even more likely influencing the liquid impermeability, e.g. by forming discontinuities or even apertures in the layer.

Direct bonding of moisture vapour permeable, liquid impermeable layers to a suitable substrate, e.g. a nonwoven layer or a microporous film in order to form an e.g. composite layered structure, is therefore preferred, and is particularly suitable for moisture vapour permeable, liquid impermeable layers formed from thermoplastic hydrophilic polymeric compositions as those referred to above. It is typically achieved by making use of the intrinsic tackiness of the thermoplastic hydrophilic polymeric composition when in molten or semi-molten or plastic state, for example by extruding a layer made of the hydrophilic polymeric composition directly onto the substrate, or by hot-melt coating, that is by coating the substrate with a layer of the hydrophilic polymeric composition typically in a low viscosity molten state. Suitable methods, particularly for hot melt coating, are for example referred to in patent applications WO 99/64077 or WO 99/64505, where hydrophilic polymeric compositions for moisture vapour permeable, liquid impermeable structures are disclosed, which have a low viscosity and are particularly suitable for hot melt coating processes. Preferred compositions and methods are described extensively in our case EP1193289.

However, although having improved mechanical properties, multilayer structures made according to EP1193289 may still have a tendency to delaminate i.e. to loose adhesion between the layers, when subjected to particularly harsh physical treatment such as repeated bending and stretching as it can occur when such multilayer structures are employed in e.g. sanitary articles such as panty liners, napkins and the like.

An alternative type of structure combining the use of a low cost substrate with good mechanical properties and stability, waterproofness and moisture vapor permeability has been suggested in US 4,613,544 which describes a microporous membrane where the pores are filled with a hydrophilic composition which is water vapor permeable and liquid impermeable.

This solution can have some advantages if compared with multi layer structures as those described above, in fact, since the hydrophilic material does not form a continuous film but limits to fill the pores, the composite structure does not have a tendency to delaminate. The downside of this method is that it requires a complex process which includes preparing a liquid composition containing the hydrophilic material, causing said liquid composition to flow only into the pores of the matrix and subsequently causing the liquid composition to convert to solid hydrophilic material. Preferred methods suggest the use of a solution of a precursor of the hydrophilic material which is then polymerized in situ or the use of a polymer solution which is infiltrated into the pores and where the solvent is then evaporated.

US 4,833,026 describes breathable and waterproof sheet materials similar to those described above comprising a microporous polymeric film and a hydrophilic filler material infiltrated into the pores of the film, and to methods for making such sheet materials. In the methods described herein, the liquid hydrophilic material or precursor thereof is infiltrated into the pores of the microporous film after the film has been stretched in the lengthwise direction, but before the film is stretched in the transverse direction. By coating the microporous film prior to the transverse stretching step, superior waterproof sheet materials are obtained. The sheets produced with this method are improved vs. of US 4,613,544, but the making process is even more complicated by an additional stretching step.

It is therefore an object of the present invention to provide improved hydrophilic polymeric compositions having unique rheological properties, and which in addition can be processed and applied, e.g. onto a substrate, using simpler production techniques, thus allowing to e.g. produce water vapour permeable, liquid impermeable composite structures by means of simple processes such as slot coating.

Another object of the present invention is to provide composite structures, comprising said improved polymeric compositions, which are water vapour permeable and liquid impermeable and, in addition, show an improved structural stability in use, particularly having no tendency to delamination under stress.

### Summary of the Invention

The present invention relates to a new hydrophilic polymeric composition comprising:
a thermoplastic hydrophilic polymer or a mixture of thermoplastic hydrophilic polymers selected from the group consisting of:
   polyurethanes, copolyurethanes or block polyurethanes and their derivatives, polyamides and co-polyamides, polyesters and copolyesters and their sulfonated derivatives, polyether and polyether copolymers, polyether-esters and polyether-ester block copolymers, polyether-amides and polyether-amide block copolymers, polyester-amides and polyester-amide block copolymers, polyether-ester-amides and polyether-ester-amide block copolymers, polyvinyl alcohol copolymers, poly-glycolic acid copolymers, poly-lactic acid copolymers, acrylic and vinylic copolymers, and mixtures thereof.
   a suitable compatible plasticiser or blend of suitable compatible plasticisers.
The hydrophilic polymeric composition having a tangent δ at 25ºC and 1 rad/sec > 0.8, preferably > 1, more preferably > 1.5,
wherein the complex viscosity η* at 25ºC and 1 rad/sec is comprised between 0.01 - 1000 Pa•s, preferably between 0.1 to 500 Pa•s, and more preferably between 0.5 - 200 Pa•s.

The hydrophilic polymeric composition of the present invention allows making a moisture vapour permeable, liquid impervious, composite material by coating said hydrophilic polymeric composition onto a suitable substrate. We have surprisingly found that layers obtained by said hydrophilic polymeric composition, does not delaminate even when the composite structure in used in harsh conditions.
This invention also relates to a moisture vapour permeable, liquid impervious composite structure comprising a microporous film coated with a polymeric composition
having a tangent δ at 25º C and 1 rad/sec > 0.8, preferably > 1, more preferably > 1.5
wherein the complex viscosity η* at 25º C and 1 rad/sec is comprised between 0.01 - 1000 Pa•s, preferably between 0.1 to 500 Pa•s, and more preferably between 0.5 - 200 Pa•s.
The composite structure has a water vapour transmission rate (WVTR) of at least 300 g/m² 24h, more preferably of at least 500 g/m² 24h, even more preferably of at least 600 g/m² 24h, most preferably of at least 1000 g/m² 24h, said water vapour transmission rate measured according to the modified ASTM E-96 "Upright Cup" Method referred to herein.

### Detailed Description of the Invention

By saying "hydrophilic polymer" or "hydrophilic polymeric composition" it is herein intended a polymer, typically a thermoplastic polymer, or a polymeric composition capable of forming a film or layer or coating that does not allow the flow of moisture vapour through open pores or apertures in the material, but does transfer substantial amounts of moisture vapour through the film or layer or coating by absorbing water on one side of the film or layer or coating where the moisture vapour concentration is higher, and desorbing or evaporating it on the opposite side of the film or layer or coating where the moisture vapour concentration is lower (monolithic films or layers or coatings, as explained herein). "Hydrophilic polymer" and "hydrophilic polymeric composition" are therefore to be considered synonymous of "monolithic polymer" and "monolithic polymeric composition" in the present description.

As it will be explained in more detail below, the hydrophilic polymeric compositions of the present invention, owing to their own selected rheology at room temperature, cannot be said to be "thermoplastic", since they are not solid at room temperature. However, an increase in temperature does cause a decrease in the viscosity of the polymeric composition of the present invention, similarly to what happens to truly thermoplastic polymeric compositions.

The terms "breathable" and "breathability" are intended herein to correspond to "moisture vapour permeable" or "water vapour permeable", and "moisture vapour permeability" or "water vapour permeability", typically referred to "monolithic compositions" and "monolithic layers or coatings or films" as defined in the Background of the Invention, as well as to composite structures comprising said monolithic compositions according to a preferred embodiment of the present invention. "Moisture vapour" and "water vapour" are also considered to be equivalent.

According to an embodiment of the present invention, the polymeric hydrophilic compositions for making moisture vapour permeable, liquid impervious structures at least comprises a thermoplastic hydrophilic polymer or a mixture of thermoplastic hydrophilic polymers, and a suitable plasticiser, or a blend of suitable plasticisers.

As it is known in the art, a plasticiser is broadly defined as an organic compound added to a polymer both to facilitate processing and, when it is solid, to increase the flexibility and toughness of the final product. As taught in our patent applications WO 99/64077 and WO 99/64505, a plasticiser or a blend of plasticisers can be included in a hydrophilic polymeric composition comprising a thermoplastic hydrophilic polymer or polymers in order to lower the viscosity of said thermoplastic hydrophilic polymer or polymers at the process conditions, which would be otherwise rather high. This facilitates the processability of these compositions, typically in order to provide a layer in a desired, and preferably low, thickness, onto a substrate, either a formation substrate for subsequent transfer on a structural substrate, or, typically, directly on a structural substrate for the formation of a composite layered structure. WO 99/64505 particularly discloses preferred plasticisers which, in addition to adjusting the viscosity of the compositions, are also capable of substantially keeping, and in some cases also increasing, the breathability of the pure thermoplastic hydrophilic polymer or polymers comprised in the compositions.
According to a preferred embodiment, the present invention discloses hydrophilic polymeric compositions for making moisture vapour permeable, liquid impermeable layers or structures, in which a specific selection of thermoplastic hydrophilic polymer or polymers, and of a suitable compatible plasticiser or blend or plasticisers provides the final composition with a selected rheology at room temperature defined by:
a tangent δ at 25º C and 1 rad/sec > 0.8, preferably > 1, more preferably > 1.5
and
a complex viscosity η* at 25º C and 1 rad/sec comprised between 0.01 - 1000 Pa•s, preferably between 0.1 to 500 Pa•s, and more preferably between 0.5 - 200 Pa•s.

The tangent δ corresponds, as it is known in the art, to the ratio G'/G" wherein G' is the elastic modulus and G" the storage modulus of the composition evaluated at certain temperature and frequency.

This selected rheology provides for a polymeric composition according to the present invention which is not solid at room temperature, but rather "creamy" or "pasty", and can be applied or coated onto a substrate by being spread, also at room temperature.

Suitable thermoplastic hydrophilic polymers according to the present invention can be selected among the thermoplastic hydrophilic polymers disclosed in our copending applications WO 99/64077, WO 99/64505, and EP 1193289. Specifically, suitable thermoplastic hydrophilic polymers according to the present invention can be selected from the group consisting of polyurethanes, copolyurethanes or block polyurethanes and their derivatives, polyamides and co-polyamides, polyesters and copolyesters and their sulfonated derivatives, polyether and polyether copolymers, polyether-esters and polyether-ester block copolymers, polyether-amides and polyether-amide block copolymers, polyester-amides and polyester-amide block copolymers, polyether-ester-amides and polyether-ester-amide block copolymers, polyvinyl alcohol copolymers, poly-glycolic acid copolymers, poly-lactic acid copolymers, acrylic and vinylic copolymers, and mixtures thereof.

Preferred are polymers selected from the group consisting of polyurethanes, copolyurethanes or block polyurethanes and their derivatives, copolyesters, polyester-amide block copolymers, polyether block copolymers, polyether-amide block copolymers, polyether-ester-amide block copolymers and polyether-ester block copolymers, and mixtures thereof.

Particularly preferred thermoplastic hydrophilic polymers are thermoplastic poly-ether-amide block copolymers (e.g. Pebax^{TM}), thermoplastic polyester block copolymers (e.g. Hytrel™), and thermoplastic polyurethanes, typically non reactive polyurethanes (e.g. Estane^{TM}), or mixtures thereof.

Suitable plasticisers according to the present invention can be selected from the group consisting of citric acid esters, tartaric acid esters, glycerol and its esters, sucrose esters, adipates, sebacates, sorbitol, epoxidized vegetal oils, polymerised vegetal oils, polyols, phthalates, liquid polyesters, glycolates, p-toluene sulfonamide and derivatives, glycols and polyglycols and their derivatives, sorbitan esters, phosphates, monocarboxylic fatty acids (C₈-C₂₂) and their derivatives, and mixtures thereof.

Preferred plasticizers are those which are liquid at 25ºC. Less preferably, plasticisers which are not liquid at 25°C, but which are liquid at 80°C, can be also selected.

According to a preferred embodiment of the present invention, a suitable plasticiser, or a blend of suitable plasticisers, and a thermoplastic hydrophilic polymer, or mixture of thermoplastic hydrophilic polymers, to be comprised in the hydrophilic polymeric composition, are preferably selected such that said suitable plasticizer, or blend of suitable plasticizers, can be absorbed into the selected thermoplastic hydrophilic polymer or mixture of thermoplastic hydrophilic polymers.

The capability of the hydrophilic polymer or mixture of hydrophilic polymers of absorbing the plasticizer or blend of plasticizers can be measured as Polymer/Plasticizer Absorption Ratio according to the Plasticizer Absorption Test described herein.

According to a preferred embodiment of the present invention, it has been found that at least 80%, preferably at least 90%, more preferably 100%, by weight of the hydrophilic polymeric composition, consists of a thermoplastic hydrophilic polymer, or of a mixture of thermoplastic hydrophilic polymers, and of a suitable compatible plasticiser, or of a blend of suitable compatible plasticisers, which are selected such that the thermoplastic hydrophilic polymer and the suitable compatible plasticiser, in case only one polymer and one plasticiser are present in the hydrophilic polymeric composition, or alternatively each polymer/plasticiser combination thereof, in case more than one thermoplastic hydrophilic polymer and/or more than one suitable compatible plasticiser are present in the hydrophilic polymeric composition, have a polymer/plasticiser absorption ratio of at least 30%, preferably of at least 40%, more preferably of at least 50%. The polymer/plasticiser absorption ratio is measured according to the Plasticiser Absorption Test described herein.

The preferred Polymer/Plasticiser Absorption Ratio of the or of each polymer/plasticiser combination in the hydrophilic polymeric composition of the present invention has to be preferably satisfied at the test temperature of 25°C. Alternatively, said preferred Polymer/Plasticiser Absorption Ratio has to be satisfied at the test temperature of 80°C, as disclosed more in detail in the Plasticiser Absorption Test described herein.

According to the present invention, it is relevant that the hydrophilic polymeric composition is mostly comprised of one or more thermoplastic hydrophilic polymers, and of one or more suitable compatible plasticisers, wherein the one or each thermoplastic hydrophilic polymer/plasticiser combination, which is/are possible among the selected polymer or polymers and plasticiser or plasticisers, satisfies the requirement of the preferred polymer/plasticiser absorption ratio. By way of example, in a hydrophilic polymeric composition according to the present invention a selected thermoplastic hydrophilic polymer can show the desired polymer/plasticiser absorption ratio towards one or more, for example two, selected suitable compatible plasticisers present in the composition itself. Preferably, the thermoplastic hydrophilic polymer and the two suitable compatible plasticisers satisfying the Polymer/Plasticizer Absorption Ratio overall shall constitute at least 80% by weight of the entire amount of the hydrophilic polymeric composition, preferably at least 90%, and more preferably constitute the entirety of the hydrophilic polymeric composition.

Without being bound to any theory, it is believed that the capability of the selected hydrophilic polymer or polymers to absorb the selected plasticizer or plasticizers at a given relatively low temperature, preferably at room temperature, as exemplified by the preferred test temperature of 25°C in the Plasticiser Absorption Test, is relevant to the purpose of achieving a hydrophilic polymeric composition which does not solidify at room conditions, but instead features a viscous pasty nature, which can be also defined as "creamy", such that it can be applied or coated onto a substrate by being actually spread, also at room temperature.

The Plasticizer Absorption Test used herein is a modification of the ASTM D 570-81 method used to measure the Water Absorption ratio in Polymers. The resulting value obtained from this test is the Polymer/Plasticizer Absorption Ratio of one or each polymer/plasticizer combination present in the composition of the present invention, which depends on the choice both of the thermoplastic hydrophilic polymer or mixture of polymers and of the suitable plasticizer or blend of plasticizers.

According to the present invention, it is preferred that the overall amount of the thermoplastic hydrophilic polymer or mixture of thermoplastic hydrophilic polymers, is comparable to the overall amount of the suitable compatible plasticiser, or blend of suitable compatible plasticisers, particularly with reference to the hydrophilic thermoplastic polymer or polymers and suitable compatible plasticiser or plasticisers which satisfy the preferred polymer/plasticiser absorption ratio as explained before.

Preferably the polymeric hydrophilic composition of the present invention comprises from 10% to 75%, preferably from 25% to 65%, more preferably from 40% to 60%, by weight of the polymeric hydrophilic composition, of the selected thermoplastic hydrophilic polymer or mixture of selected thermoplastic hydrophilic polymers, and from 25% to 90%, preferably from 35% to 75%, more preferably from 40% to 60%, by weight of the hydrophilic polymeric composition, of the selected suitable compatible plasticiser or blend of selected suitable compatible plasticisers.

The hydrophilic polymeric compositions according to the present invention can also comprise further thermoplastic polymer or polymers, and also further plasticiser or plasticisers, besides those selected according to the present invention as described above. For example further hydrophilic polymer or polymers and plasticiser or plasticisers can be selected among those disclosed in our patent applications WO 99/64077 or WO 99/64505. Preferably, these further components should not be included in an amount higher than 20% by weight of the hydrophilic polymeric composition.

Traditional known tackifying resins can also be included in the hydrophilic polymeric compositions of the present invention, if for example an enhancement of the tackiness level of the composition is desired. Suitable tackifying resins can be selected among those disclosed in our patent applications WO 99/64077 or WO 99/64505 and WO 02/28951. Preferably the total amount of tackifying or of blend of tackifying resins should be from 0 to 20%, preferably less than 15%, more preferably less than 10% by weight of said hydrophilic polymeric composition.

The hydrophilic polymeric compositions of the present invention may in addition comprise additional optional components to further improve the processability of the compositions and also the mechanical characteristics as well as other characteristics as resistance to ageing by light and oxygen, visual appearance etc., of the layers formed from such hydrophilic polymeric compositions.

In general, the hydrophilic polymeric compositions of the present invention may include anti-oxidants, anti-ultraviolets, water scavengers, hydrolytic stabilizers, pigments, dyes, odor adsorbing materials, perfumes, pharmaceuticals, drugs, medicaments, antibacterials, biocides, antiviruses, and mixtures thereof, which may be present within the compositions at a level of up to 10% by weight of the composition. The hydrophilic polymeric composition of the present invention is particularly useful to incorporate these active ingredients, since, due to its "creamy" nature, it can be processed in conventional hot melt equipment at lower temperatures compared to previously known not "creamy" hydrophilic polymeric compositions. This allows the incorporation, directly into the melt, of numerous active materials which otherwise could degrade at the processing temperatures which are typical of known hot melt and extrusion processes, such as volatile perfumes, or compounds which decompose when heated.

Further components which can be included in the hydrophilic polymeric compositions of the present invention are e.g. materials in particulate or powder form which can provide a further desired effect, such as for example any known material capable of providing odour control, e.g. zeolites, or any known active agent release material, or known fluid absorbent materials as, for example, a superabsorbent material, and so on. In case a superabsorbent material is added to provide the hydrophilic polymeric composition of the present invention with a water absorption capacity, the average particle size of the particles of said superabsorbent material for use herein is in dry state from 0.1 µm to 500 µm, preferably from 1 µm to 200 µm, more preferably from 10 µm to 100 µm, even more preferably from 10 µm to 60 µm and most preferably from 15 µm to 40 µm. Without the intention to be bound by theory it is believed that the smaller the average diameter of the particles of superabsorbent material are, the better their absorbency towards liquids is. Indeed the effective surface area, which is in contact with the liquid to be absorbed, is much larger for a large quantity of small particles compared to a smaller quantity of larger particles of the same overall weight. In general, a particle size of the superabsorbent particles of smaller than 0.1 µm may result in process problems, as by the very fine particles the generation of dust while handling those particles, e.g. during manufacture of the composition of the present invention, is highly likely. On the other hand, when using superabsorbent particles being larger in diameter than 500 µm, it is not possible anymore to provide thin layers of the composition of the present invention. The minimum thickness of such a layer is determined by the diameter of the superabsorbent particles incorporated therein.

According to the present invention a moisture vapour permeable, liquid impervious composite material can be formed from the hydrophilic polymeric compositions of the present invention, by laying said hydrophilic polymeric composition onto a suitable substrate. The compositions of the present invention can provide composite materials which are liquid impermeable and also have a good level of moisture vapour permeability (breathability), similar to that which would be obtained by forming on said substrate a solid film or layer having the same thickness and comprising only the same pure thermoplastic hydrophilic polymer or polymers, or the same thermoplastic hydrophilic polymer or polymers and a different selection of plasticiser or plasticisers not providing the desired rheological properties of the composition of the present invention. Particularly preferred compositions according to the present invention can also provide composite materials having higher levels of breathability when compared to composite materials obtained forming on said substrate a solid film or layer having the same thickness and comprising only the same pure thermoplastic hydrophilic polymer or polymers. According to this particularly preferred embodiment of the present invention, a suitable compatible plasticiser or blend of plasticisers satisfying the preferred Polymer/Plasticiser Absorption Ratio can be selected among the preferred hydrophilic plasticisers described in our copending application WO 99/64505, and among glycols and polyglycols.

The composite materials comprising the hydrophilic polymeric compositions of the present invention preferably have a moisture vapour transmission rate of at least 300 g/m² 24h, more preferably of at least 500 g/m² 24h, even more preferably of at least 600 g/m² 24h, most preferably of at least 1000 g/m² 24h, said water vapour transmission rate measured according to the modified ASTM E-96 "Upright Cup" Method. Preferably said rates are achieved with a thickness of a typically continuous layer of hydrophilic polymeric composition of at least 20 µm,.

According to the present invention, layers can be formed from the hydrophilic polymeric compositions described so far which have a thickness of from about 0.2 µm to about 200 µm and above, said layers can be coated on different substrates, such as for example a woven or nonwoven fibrous material or a microporous film.

Layers or coatings of the compositions of the present invention can be made according to known methods as will be explained more in detail hereinbelow.

Layers or coatings made according to present invention are not necessarily continuous, i.e. liquid impervious, in fact, if such a layer is coated onto a substrate which is only slightly water permeable, such as for example a microporous film, they can form in combination a composite structure which is moisture vapor permeable and liquid impervious, even if each single component, i.e. the layer of the hydrophilic polymeric composition of the present invention and the microporous film substrate, is not perfectly liquid impervious per se.

According to a preferred embodiment the present invention, the suitable compatible plasticiser or plasticisers selected as described above in combination with the thermoplastic hydrophilic polymer or polymers, in addition to providing the final hydrophilic polymeric composition with the desired rheological properties at room temperature, are also preferably effective in increasing the processability of the hydrophilic polymeric compositions of the present invention, typically by adjusting the viscosity of said compositions at the process conditions, in a similar way as already disclosed in our patent applications WO 99/64077 or WO 99/64505.

While in fact the hydrophilic polymeric composition of the present invention, by being creamy or pasty, i.e. not solid, at room temperature, can be in principle applied onto a suitable substrate at room temperature, it is preferred that a suitable increase of the process temperature is achieved, in order to better adjust the viscosity at these conditions.
The hydrophilic polymeric compositions of the present invention will preferably have the following complex viscosities (η*) at the process conditions, such that they can be applied onto the selected substrate using known hot melt coating techniques while then having the desired "creamy" state after cooling at room conditions:

50 poise < η* < 4000 poise, preferably 100 poise < η* < 2000 poise, more preferably 100 poise < η* < 1000 poise, at a frequency of 1 rad/s at a temperature of 210°C or less and η* < 2000 pose, preferably η* < 1000 poise, more preferably η* < 500 poise, at a frequency of 1000 rad/s at a process temperature (T) of 210°C or less, wherein η* represents the complex viscosity of the thermoplastic polymeric hydrophilic composition. Preferably the temperature T is 200°C or less and more preferably 180°C or less and most preferably from 150°C to 50°C.

According to this preferred embodiment of the present invention the hydrophilic polymeric compositions having the complex viscosity described above allow for a coating or layer to be coated onto a substrate using typical coating conditions and apparatuses known in the art for the coating of low viscosity hot melt compositions in a layer having a required thickness onto a substrate, but preferably at lower temperature compared to those typically known in hot melt processes, while also keeping the advantageous characteristics of the hydrophilic polymeric compositions of the present invention in providing a composition having the desired "creamy" state at room conditions, i.e., without setting into a solid film or layer.

Hydrophilic polymeric compositions according to the present invention having such viscosities can also provide very thin layers.

A hydrophilic polymeric composition according to the present invention can be manufactured with any known process that will typically comprise the steps of providing at least the selected thermoplastic hydrophilic polymer or mixture of polymers and the selected suitable compatible plasticiser or blend of plasticisers, and optionally any further additional components as explained above, such as for example a tackifier or blend of tackifiers, heating the components and compounding them, e.g. with a known suitable mixer to form the hydrophilic polymeric composition in the molten state for subsequent process steps.

Alternatively, solvent or emulsion systems can be created and used to process the hydrophilic polymeric compositions of the present invention, either as intermediate or final step in making moisture vapour permeable, liquid impermeable structures from said compositions, and articles comprising said structures.

A preferred process for making a layer from a polymeric hydrophilic composition according to the present invention typically comprises the steps of providing said composition, preferably heating it to make it more flowable to the extent that a hot melt coating process can be used for forming said composition onto a substrate in a layer having the desired thickness.

In principle the composition of the present invention can be directly applied onto the desired substrate at room conditions, taking advantage of its viscous, "creamy" state. Of course a suitable heating can further lower the viscosity of the composition at the process conditions, allowing the use of a simple hot melt coating process.

In a preferred embodiment of the present invention a moisture vapour permeable, water impervious composite structure can be formed which comprises the hydrophilic polymeric composition and a suitable substrate onto which said hydrophilic polymeric composition is laid, wherein the substrate is also preferably moisture vapour permeable. The rheological properties imparted to the hydrophilic polymeric composition of the present invention by the disclosed selection of the thermoplastic hydrophilic polymer or polymers, and of the suitable compatible plasticiser or plasticisers, typically provide for an increased resistance to delamination in use for the resulting composite structure, also with very thin layers of the hydrophilic polymeric composition. The better resistance to delamination is due to the fact that the composition does not set or solidify at room conditions into a solid film, which may have a greater tendency to separate from the substrate (i.e. delaminate) under particularly critical and stressing usage conditions. The hydrophilic polymeric composition of the present invention rather forms a soft and flexible, highly viscous layer which can more effectively withstand mechanical stresses in use. Said better resistance of the resulting composite structure is combined with no detrimental effect at all on the water vapour transmission capability of the layer formed from the hydrophilic polymeric composition of the present invention, for example if compared to a solid layer or film of the same thickness formed from a similar composition, but not having the desired rheological properties as described herein by not comprising the selected combination of polymer or mixture of polymers and of compatible plasticiser or blend of plasticisers according to the present invention. Particularly preferred plasticisers according to the present invention can also increase the water vapour transmission rate of a layer formed from the hydrophilic polymeric composition of the present invention, when compared to a layer of the same thickness formed from a similar composition. Suitable substrates to be used in said composite structures are, for example a fibrous substrate such as a nonwoven layer or, preferably, a microporous film.

Other known processes can be used for making moisture vapour permeable, liquid impermeable structures, not limited to coatings and layers, from the hydrophilic polymeric compositions of the present invention, and articles comprising said structures.

### Preparation methods

A composite structure can be produced with two or more layers where at least one of the layers comprises the hydrophilic polymeric composition of this invention. This can be accomplished by a variety of known means, including but not limited to: co-extrusion, extrusion coating, etc. A preferred method is hot melt coating. Such a structure can be included into an article.

The structure or the article can be a composite with one or more other materials, the composite can involve at least one component of the hydrophilic polymeric composition in combination with one or more other materials. Such materials include, but are not limited to: fibres, fibrous batts, non-wovens, wovens, papers, micro-porous or porous films or membranes, films such as polymeric films, inorganic structures such as compressed gypsum sheets, perforated or apertured films and papers, macroscopically expanded films, cloth, substantially rigid fibre-based materials such as lumber, etc.

Said other components may be non-absorbent, absorbent, liquid containing, etc.

According to a preferred embodiment of the present invention, a composite structure comprising the hydrophilic polymeric composition coated into a layer on a microporous film can be provided.

An alternative useful technique is the process of spray coating. The hydrophilic polymeric composition of this invention lends itself to a heated spraying technique whereas upon heating the viscosity is sufficiently lowered to allow spray coating or sputtering onto a suitable substrate. Alternately, the spray coating method can employ different starting raw material formats of the hydrophilic polymeric composition such as a solvent-based approach or an emulsion.

For a composite article comprising the hydrophilic polymeric composition of the present invention, and employing the spray coating approach, the other material may provide sufficient three dimensional structure by itself such that the other material acts as a mould, after which it is sufficiently coated the composite article is complete.

In an embodiment of the present invention a moisture vapour permeable, liquid impervious composite layered structure is typically provided wherein the contribution of the layer formed from the hydrophilic polymeric composition of the present invention to the overall performance of the composite material resides in the provision of a breathable liquid barrier and hence can be advantageously provided as thinly as possible. The remaining performance physical criterion is then typically provided by the provided substrate, which therefore acts also as a support layer.

The substrate, or support layer may be any useful layer which is preferably also moisture vapour permeable, preferably having a moisture vapour permeability of at least 100 g/m² 24h, more preferably at least 300 g/m² 24h, and most preferably at least 500 g/m² 24h.

Suitable substrates for use herein as support layers in composite structures include two dimensional, planar micro and macro-porous films; macroscopically expanded films; formed apertured films; nonwoven and woven layers. According to the present invention the apertures in said layer may be of any configuration, but are preferably spherical or oblong and may also be of varying dimensions. The apertures preferably are evenly distributed across the entire surface of the layer, however layers having only certain regions of the surface having apertures are also envisioned.

Suitable two dimensional porous planar layers may be made of any material known in the art, but are preferably manufactured from commonly available polymeric materials. Suitable materials are for example Goretex™ or Sympatex^{TM} type materials well known in the art for their application in so-called breathable clothing. Other suitable materials include XMP-1001 of Minnesota Mining and Manufacturing Company, St. Paul, Minnesota, USA and Exxaire XBF-101W, supplied by the Exxon Chemical Company. As used herein the term two dimensional planar layer refers to layers having a depth of less than 1 mm, preferably less than 0.5 mm, wherein the apertures have an average uniform diameter along their length and which do not protrude out of the plane of the layer. The apertured materials for use in the present invention may be produced using any of the methods known in the art such as described in EP 293 482 and the references therein.

According to the present invention, particularly preferred support layers for use herein include planar microporous films.

Suitable apertured formed films include films which have discrete apertures which extend beyond the horizontal plane of the surface of the layer thereby forming protuberances. The protuberances have an orifice located at its terminating end. Preferably said protuberances are of a funnel shape, similar to those described in US 3,929,135. The apertures located within the plane and the orifices located at the terminating end of protuberance themselves maybe circular or non circular provided the cross sectional dimension or area of the orifice at the termination of the protuberance is smaller than the cross sectional dimension or area of the aperture located within the surface of the layer. Preferably said apertured preformed films are unidirectional such that they have at least substantially, if not complete one directional fluid transport.

Suitable macroscopically expanded films for use herein include films as described in for example in US 4,637,819 and US 4,591,523.

The composite layered structures of this preferred embodiment of the present invention are particularly advantageous as they allow the possibility of providing a composite wherein the thermoplastic composition may be formed onto the support substrate as a layer or coating with the desired thickness. By e.g. suitably tailoring the viscosity of the hydrophilic polymeric composition at the process conditions as explained above, typical coating conditions and apparatuses known in the art for the direct coating of low viscosity hot melts can be readily utilized in order to provide the hydrophilic polymeric composition at the desired thickness onto the substrate.

The hydrophilic polymeric compositions for making moisture vapour permeable, liquid impermeable structures according to the present invention have been so far described as being provided with a desired tackiness typically in the low viscosity state at the process conditions. This is desired in a preferred embodiment of the present invention in order to form e.g. more stable moisture vapour permeable, liquid impermeable layered composite structures with the hydrophilic polymeric composition directly formed as a coating or layer onto a suitable substrate, wherein said tackiness to a substrate is not achieved to the detriment of the moisture vapour permeability of the resulting layer or film. Of course this corresponds to a better adherence of the hydrophilic polymeric composition to the selected substrate at the coating stage, although no true adhesive behavior is actually developed, since the compositions do not set into a solid at room temperature, but remains creamy as explained above.

According to another embodiment of the present invention, a moisture vapour permeable, liquid impervious composite structure is provided which structure comprises a microporous film coated with a polymeric composition which has
a tangent δ at 25º C and 1 rad/sec > 0.8, preferably > 1, more preferably > 1.5
and
a complex viscosity η* at 25º C and 1 rad/sec comprised between 0.01 - 1000 Pa•s, preferably between 0.1 Pa•s to 500 Pa•s, and more preferably between 0.5 - 200 Pa•s.

The composite structure according to the present embodiment has a water vapour transmission rate (WVTR) of at least 300 g/m² 24h, more preferably of at least 500 g/m² 24h, even more preferably of at least 600 g/m² 24h, most preferably of at least 1000 g/m² 24h. The water vapour transmission rate is measured according to the modified ASTM E-96 "Upright Cup" Method referred to herein.

In this alternative embodiment of the present invention, the polymeric composition can typically comprise a thermoplastic polymer, or a mixture of thermoplastic polymers which can be not intrinsically hydrophilic per se, or less hydrophilic than the selected polymers of the preferred embodiment of the present invention, and a suitable compatible plasticizer or blend of suitable compatible plasticizers. The plasticizer or plasticizers can be suitably selected among those already mentioned with reference to the preferred embodiment of the present invention. The preferred conditions of the Polymer/Plasticizer Absorption Ratio, as previously described with reference to the thermoplastic hydrophilic polymeric composition of the preferred embodiment of the present invention, also preferably apply to this alternative embodiment.

Typically breathability of the composite structure of this alternative embodiment can be enhanced by a coating of the polymeric composition which is as thin as possible, and preferably not continuous, provided the liquid imperviousness of the overall composite structure is however achieved.

### USES

The preferably hydrophilic polymeric compositions of the present invention and the moisture vapour permeable, liquid impervious structures, e.g. composites structures formed therefrom, find utility in a number of applications wherein liquid imperviousness and moisture vapour permeability are desirable.

Said structures can be typically disposable, e.g. structures with a nonwoven as a substrate, or alternatively durable or semi-durable, such as for example laminated structures comprising a textile or a fabric as a substrate or support.

In particular the present invention can be effectively utilised within personal care products, such as absorbent articles, wound care articles, or cosmetics. Non limiting examples are absorbent articles such as diapers, sanitary napkins, panty liners, incontinence products and breast pads; wound and burn dressings and bandages, warming or cooling pads for medical use; patches, bandages or wraps, e.g. for medical or cosmetic treatment, which may contain and deliver active substances; perspiration pads such as underarm-, wrist- and head perspiration pads, collar inserts, shoe inserts, hat bands; cosmetics such as make up, face masks, lipsticks, or hair gels, in order to create on the skin or on the hair a breathable coating, nail polish, etc.

Other articles comprising the preferably hydrophilic polymeric compositions of the present invention comprise protective articles for the body, or for body parts. Non limiting examples comprise protective clothing such as working or surgical gowns and the like; hand coverings such as gloves, finger cots, mitts, mittens; foot or leg coverings such as socks, hose, pantyhose, shoes, slippers; head coverings such as hats, caps; prophylactic and contraceptive mechanical articles such as condoms; face coverings such as face masks, nose covers, ear covers or mitts; sport and fitness wearing articles, wind cheaters, sleeping bags; body support items such as male organ "athletic" supporters, brassieres; clothing for use as underwear, protective sleeves, or as a part of or wholly incorporated into protective pads. Other example articles and applications include but are not limited to: flexible or drapable clothing articles for humans such as the non-limiting examples of shirts, pants, undergarments, bibs, smocks, coats, scarves, body wraps, stockings, leggings, skirts, dresses, etc.; other flexible or drapable clothing or protecting sheets for various tasks and occupations including medical professions, agricultural jobs, mechanical assembly and repair, emergency public services, the military, athletic endeavors, cleaning positions; protective garments for animals.

A further category of articles comprising the preferably hydrophilic polymeric compositions of the present invention comprises articles for protecting objects. Preferred protecting articles comprise protecting bedding covers such as linen, mattress and pillow covers. Also protecting covers for cushions, comforter, duvets, upholstered portions of beds, such as headboards, or of sofas or armchairs are comprised. Other non limiting examples comprise protective articles such as dust covers for electronic/electrical products (e.g. computer keyboards, hard drives, video recorders, etc.), headrest covers for seats in vehicles, e.g. airplanes/trains, shrink wraps, one use table covers, etc. Articles for packaging such as for food products such as fresh produce and baked goods (bread, rolls, cakes), e.g. bags for food storage in the refrigerator, or also packaging films for microwave oven, or packages for hot "take away" foods, e.g. pizza. Further examples comprise articles for agriculture and horticulture such as, as non-limiting examples, an individual article (container, three dimensional "bag") which is placed to partially or totally enclose an individual or specific group of plants. Protective furniture coverings such as protective covers for upholstered chairs and sofas, etc. are also comprised. Other alternative protecting articles comprise construction roofing materials and house wrapping, ski, windsurf and bike/motor bike overalls, backings for carpets and wallpapers, camping tents, protecting sheets for various items (e.g. cars, tennis courts, sport grounds, etc.), sheets for gardens/greenhouses protection, tents for closing/protecting tennis courts, sport grounds, items for protection of plants from low temperatures, etc.

Alternative applications in which the preferably hydrophilic polymeric compositions of the present invention are applied via spraying/brushing/roll coating, typically in the form of a solvent or emulsion based composition and at room temperature, comprise protective coatings for hard surfaces such as stone, concrete, wood (e.g. furniture), for coating/water proofing of shoes/leather articles or textiles, protective coatings for cars (e.g. during transport by ship), protective coatings for cars, boats etc. during long periods of non use. The preferably hydrophilic polymeric compositions of the present invention can also be comprised in breathable paints.

More in general, whenever possible in the many different applications mentioned above, the preferably hydrophilic polymeric compositions of the present invention can be either provided as an already formed layer on a substrate or structure, or alternatively also applied in liquid or semi liquid form, e.g. sprayed or brushed, and also possibly comprising active agents, for example to the body, e.g. in a cosmetic, medical, or protective composition, or to plants.

In general all articles comprising the preferably hydrophilic polymeric compositions of the present invention can be generally flexible or rigid.

All the above articles can also comprise the hydrophilic polymeric compositions described in the already mentioned applications WO 99/64077, WO 99/64505, and EP 1193289.

A moisture vapour permeable, liquid impervious composite structure formed by forming the hydrophilic polymeric composition of the present invention onto a suitable substrate finds particular utility as the backsheet for disposable absorbent articles, especially sanitary napkins and panty liners, but also diapers, incontinence products and breast pads. Also bedding articles like mattress or pillow covers advantageously comprise the composite structure of the present invention. Such articles will typically comprise components known to the skilled person such as a liquid pervious topsheet, an absorbent core and a backsheet and may optionally also comprise other components such as fastening means, wings, and the like. A backsheet or a bedding article made of a composite structure according to the present invention provides liquid imperviousness and breathability, and, at the same time, has an improved structural stability, since it resists to delamination also in hard usage conditions.

### EXAMPLES

### Example 1 : A hydrophilic polymeric composition comprising:

| | |
|---|---|
| Estane T541 0 | 50% |
| Polyethyleneglycol 400 | 50% |
| | |
| Polymer/Plasticizer Absorption Ratio | 40% |
| Complex viscosity at 25ºC and 1 rad/sec | 5 Pa•s |
| Tangent delta at 25ºC | 3.5 |

### Example 2: Composite material

A thin coating of the composition of Example 1 (5gsm) is applied onto the microporous film Tredegar, XBF 610w.
The composite material obtained has a MVTR of 900 g/m² 24h.

### Example 3: Composite material

A thin coating of the composition of Example 1 (5gsm) is applied onto the microporous film Tredegar, XBF 616.
The composite material obtained has a MVTR of 900 g/m² 24h.

### Example 4: Article

A surgical gown material is constructed assembling the composite material of example 2 between 2 layers of a Spunbond-Meltblown-Spunbond nonwoven having a basis weight of 24gsm (from BBA Cie).

### Test methods.

### Plasticizer Absorption Test

The determination of the polymer/plasticizer absorption ratio of a thermoplastic polymer, is conducted according to the standard test method ASTM D 570-81 modified in that where said standard test method is intended to measure the water absorption of polymers, here it will be used to measure the plasticizer absorption of a polymer, thus using the selected plasticizer where the tests indicates the use of water.

The measurement of Polymer/Plasticizer Absorption Ratio for thermoplastic polymer is made on the polymer in pellet form, with pellets having a diameter ranging from 3 mm to 5 mm.

The measurement of the Polymer/Plasticizer Absorption Ratio must be run with the plasticizer in liquid form. For those plasticizers which are liquid at 25ºC the test will be run at 25ºC; for those plasticizers which are solid at 25ºC the test will be run at 80ºC. This temperature will be referred to as the "temperature of the test". Plasticizers which are not liquid at 80ºC are considered to be not suitable for the thermoplastic hydrophilic composition of the present invention, and the respective Polymer/Plasticizer Absorption Ratio in a selected thermoplastic polymer is conventionally taken as zero. Polymer/plasticiser combinations in which the polymer completely dissolves into the plasticiser at the test temperature are also considered to be not suitable for the thermoplastic hydrophilic compositions of the present invention.

According to this method 10 grams of the polymer in pellet form are immersed in 30 grams of liquid plasticizer at the temperature of the test, for 24 hours. After that, the excess plasticizer not absorbed by the polymer is separated from the pellets of polymer with known suitable means. If the temperature of the test was 80ºC the pellets are then slowly cooled at 25ºC. Finally the percentage of plasticizer absorbed is measured in accordance with the ASTM D 570-81 standard. This is called the Polymer/Plasticizer Absorption Ratio and is expressed in weight percent.

When starting from an article comprising the polymeric composition of the present invention, for example a disposable absorbent article with the polymeric composition coated onto a substrate, the polymeric composition can be isolated with known means in order to determine the polymer of mixture of polymers and the plasticizer of mixture of plasticizers contained therein. Typically in a disposable absorbent article the topsheet is removed from the backsheet and both are separated from any additional layers if present. The "creamy" polymeric composition is scraped with a spatula from its substrate layer. The recovered polymeric material can be analyzed using conventional analytical techniques well known to those skilled in the art in order to determine the polymer or mixture of polymers and the plasticizer of mixture of plasticizers contained therein. Once this has been determined the or each thermoplastic polymer shall be provided in pellet form and the plasticizer or plasticizers shall be provided in liquid form in order to run the Plasticizer Absorption Test as described herein.

### Moisture Vapour Transmission Test

Breathability of the materials of the present invention is intended to be measured as Moisture Vapour Transmission Rate at 25°C and 55% relative humidity according to the modified ASTM E-96 "Upright Cup" method. The only modification to the standard ASTM E-96 "Upright Cup" method consists in a change in the height of the air gap between the sample and the water surface in the cup, which height is 4 mm ± 0.5 mm, instead of 19 mm ± 2.5 mm, as specified in the standard test method.

### Complex Viscosity measurement

According to the present invention the complex viscosity η* and the tangent δ are measured using a Rheometer RDA-II available from Rheometrics Co.

To run complex viscosity and tangent δ measurements when starting from an article comprising the polymeric composition of the present invention, for example a disposable absorbent article with the polymeric composition coated onto a substrate, the polymeric composition can be isolated with known means in order to be tested. Typically in a disposable absorbent article the topsheet is removed from the backsheet and both are separated from any additional layers if present. The "creamy" polymeric composition is scraped with a spatula from its substrate layer. The recovered polymeric material will be used to prepare samples as mentioned above with known means.

## Claims

**1.** A hydrophilic polymeric composition comprising:
a thermoplastic hydrophilic polymer or a mixture of thermoplastic hydrophilic polymers selected from the group consisting of:
polyurethanes, copolyurethanes or block polyurethanes and their derivatives, polyamides and co-polyamides, polyesters and copolyesters and their sulfonated derivatives, polyether and polyether. copolymers, polyether-esters and polyether-ester block copolymers, polyether-amides and polyether-amide block copolymers, polyester-amides and polyester-amide block copolymers, polyether-ester-amides and polyether-ester-amide block copolymers, polyvinyl alcohol copolymers, poly-glycolic acid copolymers, poly-lactic acid copolymers, acrylic and vinylic copolymers, and mixtures thereof.
a suitable compatible plasticiser or blend of suitable compatible plasticisers,
said hydrophilic polymeric composition having a tangent δ at 25º C and 1 rad/sec > 0.8, preferably > 1, more preferably > 1.5,
wherein the complex viscosity η* at 25º C and 1 rad/sec is comprised between 0.01 - 1000 Pa•s, preferably between 0.1 to 500 Pa•s, and more preferably between 0.5 - 200 Pa•s.

**2.** A hydrophilic polymeric composition according to claim 1 wherein said thermoplastic hydrophilic polymer is selected from the group consisting of:
polyurethanes, copolyurethanes or block polyurethanes and their derivatives, copolyesters, polyester-amide block copolymers, polyether block copolymers, polyether-amide block copolymers, polyether-ester-amide block copolymers and polyether-ester block copolymers, and mixtures thereof.

**3.** A hydrophilic polymeric composition according to any of the preceding claim, wherein said suitable compatible plasticiser or blend of suitable compatible plasticisers is selected from the group consisting of:
citric acid esters, tartaric acid esters, glycerol and its esters, sucrose esters, adipates, sebacates, sorbitol, epoxidized vegetal oils, polymerised vegetal oils, polyols, phthalates, liquid polyesters, glycolates, p-toluene sulfonamide and derivatives, glycols and polyglycols and their derivatives, sorbitan esters, phosphates, monocarboxylic fatty acids (C₈-C₂₂) and their derivatives, and mixtures thereof.

**4.** A hydrophilic polymeric composition according to any of the preceding claims, **characterized in that** said thermoplastic hydrophilic polymer or mixture of thermoplastic hydrophilic polymers, and said suitable compatible plasticiser or blend of suitable compatible plasticisers, are selected such that at least 80%, preferably at least 90%, more preferably 100%, by weight of said hydrophilic polymeric composition, consists of a thermoplastic hydrophilic polymer, or of a mixture of thermoplastic hydrophilic polymers, and of a suitable compatible plasticiser, or blend of suitable compatible plasticisers, wherein said thermoplastic hydrophilic polymer and said suitable compatible plasticiser, or alternatively each thermoplastic hydrophilic polymer/plasticiser combination thereof, has a polymer/plasticiser absorption ratio of at least 30%, preferably of at least 40%, more preferably of at least 50%, said ratio measured according to the Plasticiser Absorption Test described herein.

**5.** A hydrophilic polymeric composition according to any preceding claim which comprises:
from 10% to 75%, preferably from 25% to 65%, and more preferably from 40% to 60% by weight of said hydrophilic polymeric composition, of said thermoplastic hydrophilic polymer or mixture of thermoplastic hydrophilic polymers,
from 25% to 90%, preferably from 35% to 75%, and more preferably from 40% to 60% by weight of said hydrophilic polymeric composition, of said suitable compatible plasticiser or blend of suitable compatible plasticisers,
from 0 to 20% preferably less than 15% more preferably less than 10% by weight of a suitable compatible tackifying resin or blend of suitable compatible tackifying resins.

**6.** A moisture vapour permeable, liquid impervious composite structure comprising a layer formed from the hydrophilic polymeric composition according to any of the preceding claims coated onto a moisture vapor permeable substrate.

**7.** A moisture vapour permeable, liquid impervious composite structure according to claim 6 wherein said layer of said hydrophilic polymeric composition is coated in a thickness of less than 50 microns, preferably less than 20 microns, more preferably from 0.5 to 10 microns.

**8.** A moisture vapour permeable, liquid impervious composite structure according to claim 6 or 7 wherein said composite structure has a water vapour transmission rate (WVTR) of at least 300 g/m² 24h, more preferably of at least 500 g/m² 24h, even more preferably of at least 600 g/m² 24h, most preferably of at least 1000 g/m² 24h, said water vapour transmission rate measured according to the modified ASTM E-96 "Upright Cup" Method referred to herein.

**9.** A moisture vapour permeable, liquid impervious composite structure according to claims 6 to 8, wherein said moisture vapor permeable substrate is a microporous film.

**10.** An absorbent article comprising a moisture vapour permeable, liquid impervious composite according to claims 6 to 9.

**11.** A process for making a hydrophilic polymeric composition according to claims 1 to 5, comprising the steps of:
• providing said thermoplastic hydrophilic polymer or mixture of polymers,
• providing said suitable compatible plasticiser or blend of suitable compatible plasticisers,
• heating said thermoplastic hydrophilic polymer or mixture of polymers and said suitable compatible plasticiser or blend of plasticisers and compounding them to form said thermoplastic composition in the molten state.

**12.** A process for making a composite material comprising the hydrophilic polymeric composition of claims 1 to 5, comprising the steps of:
• providing said hydrophilic polymeric composition,
• preferably heating said hydrophilic polymeric composition,
• coating said thermoplastic composition onto a substrate in a layer having a desired thickness.

**13.** A moisture vapour permeable, liquid impervious composite structure comprising a microporous film coated with a polymeric composition
said polymeric composition having a tangent δ at 25º C and 1 rad/sec > 0.8, preferably > 1, more preferably > 1.5
wherein the complex viscosity η* at 25º C and 1 rad/sec is comprised between 0.01 - 1000 Pa•s, preferably between 0.1 to 500 Pa•s, and more preferably between 0.5 - 200 Pa•s.
Said composite structure having a water vapour transmission rate (WVTR) of at least 300 g/m² 24h, more preferably of at least 500 g/m² 24h, even more preferably of at least 600 g/m² 24h, most preferably of at least 1000 g/m² 24h, said water vapour transmission rate measured according to the modified ASTM E-96 "Upright Cup" Method referred to herein.

**14.** An absorbent article comprising a moisture vapour permeable, liquid impervious composite according to claim 13.

**16.** An absorbent article according to claims 10 or 14, wherein said article is a disposable article for feminine protection, preferably a sanitary napkin or panty liner.
